# EUROPEAN PATENT APPLICATION

(11) **EP 4 767 935 A1**
(43) Date of publication of application: **01.07.2026**
(21) Application number: 24893350.9
(22) Date of filing: 15.11.2024
(51) Int. Cl.: A61B 5/145, A61B 5/00, A61B 5/1486

(54) **NEEDLE-ASSISTING DEVICE, IMPLANTABLE ANALYTE MONITORING DEVICE, AND IMPLANTABLE ANALYTE MONITORING SYSTEM**

(30) Priority: 20.11.2023 CN 202323128737 U; 30.11.2023 CN 202323240976 U
(71) Applicant: Eaglenos Sciences, Inc., Nanjing, Jiangsu 210044 (CN)
(72) Inventor: HE, Liangmao, Nanjing, Jiangsu 210044 (CN); PENG, Zhiyuan, Nanjing, Jiangsu 210044 (CN); CAI, Ya, Nanjing, Jiangsu 210044 (CN); ZHANG, Jiancheng, Nanjing, Jiangsu 210044 (CN); WANG, Jia, Nanjing, Jiangsu 210044 (CN)
(74) Representative: Regimbeau
(86) International application number: PCT/CN2024/132373
(87) International publication number: WO 2025/108192

(57) **Abstract**

Provided are an insertion device, an implantable analyte monitoring device, and an implantable analyte monitoring system. The insertion device includes a housing assembly, a needle-advancing bracket and a fixing plate that are disposed in the housing assembly, and an insertion needle assembly. The needle-advancing bracket includes a sleeve, an accommodating slot, and at least two elastic claws disposed in the sleeve. A slot opening of the accommodating slot and a sleeve opening of the sleeve face away from each other. The accommodating slot is configured to accommodate a sensor assembly. The needle-advancing bracket is disposed in the housing assembly. The needle-advancing bracket is movable along a first direction and is capable of being limited in a first position and a second position in the housing assembly. The actuation button on the housing assembly is capable of separating the housing assembly from the needle-advancing bracket. A through hole is provided at the bottom of the sleeve. The fixing plate is capable of being locked to the at least two elastic claws, and the housing assembly is capable of separating the fixing plate from the at least two elastic claws. The insertion needle assembly includes a needle hub and an insertion needle connected to the needle hub. The needle hub is circumferentially clamped between the at least two elastic claws and is disposed between the at least two elastic claws and the bottom of the sleeve. The needle hub is movable toward the fixing plate.

## Description

This application claims priority to Chinese Patent Application No. 202323128737.5 filed with the China National Intellectual Property Administration (CNIPA) on Nov. 20, 2023 and Chinese Patent Application No. 202323240976.X filed with the CNIPA on Nov. 30, 2023, the disclosures of which are incorporated herein by reference in their entireties.

### TECHNICAL FIELD

The present application relates to the technical field of medical instruments, for example, an insertion device, an implantable analyte monitoring device, and an implantable analyte monitoring system.

### BACKGROUND

With the development of society and economy and the advancement of science and technology, technologies related to medical devices gradually evolve toward intelligence and informatization. Implantable analyte monitoring, such as a continuous glucose monitoring (CGM) system, has gradually received extensive attention in the relevant fields due to its important role in blood glucose control for diabetics.

An implantable analyte monitoring system includes an implantable analyte monitoring device and an insertion device.

The implantable analyte monitoring device is configured to be implanted beneath a skin surface to obtain target analyte data from a human body. The implantable analyte monitoring device includes a sensor and a transmitter. One end of the sensor is implanted into a subcutaneous tissue of the human body, configured to react with glucose in the interstitial fluid and generate an electric signal. The other end of the sensor is connected to the transmitter and configured to transmit the electric signal to a signal processing module. A connection end of the sensor to the transmitter needs to be mounted and fixed and implements the transmission of the electric signal. The implantable analyte monitoring device may be continuously worn on the human body for 7 to 14 days and continuously monitor variations in the blood glucose level of the human body. To ensure that the user's work, life, and exercise are not affected when the user wears the device, the sensor needs to be effectively fixed so that it is ensured that the sensor can stably collect biological signals and transmit the signals to the transmitter.

In the related art, a clamping spring plate is used to fix the sensor and implement the transmission of the electric signal. However, with such a fixing and connection manner, the clamping spring contact may experience fatigue after long-time use. It is less stable to rely solely on the elasticity of the clamping spring contact to clamp and fix the sensor and maintain the signal transmission with the sensor. Particularly, after exercise or long-time use, poor contact may occur. This may result in the incapability of the transmitter to stably receive and process the electric signal, affecting the service life of the implantable analyte monitoring device.

Additionally, during the production of the implantable analyte monitoring device, the insertion needle and the implant portion of the sensor that enter the subcutaneous tissue of the human body need to be sealed through sterilization components. Thus, the implant portion of the sensor and the insertion needle can undergo sterilization and remain sterile prior to use, thereby preventing bacterial infection during use. However, how to quickly and effectively remove a needle cap used during sterilization is a problem that needs to be solved.

Implantation of the implantable analyte monitoring device into the subcutaneous tissue of the human body requires the aid of an insertion device (also referred to as an implant device). At present, an implant device, such as the medical device inserter disclosed in patent CN102573616B, includes a sheath, a device support movable between a proximal position and a distal position, a sharp support movable between the proximal position and the distal position, a handle movable between the proximal position and the distal position, and a driver. This insertion device is operated with one hand. An external force applied to a housing by the hand implants an insertion electrode of a sensor into the subcutaneous tissue of the human body through an insertion needle. Simultaneously, a spring inside the housing is compressed to store energy. The spring that stores the energy rebounds, causing the insertion needle to retract from the skin. Implant devices in the related art suffer from problems such as complex structures and inconvenience of use.

### SUMMARY

The present application provides an insertion device, an implantable analyte monitoring device, and an implantable analyte monitoring system. The structure of the insertion device is simplified. The stability of the implantable analyte monitoring device is improved. The implantable analyte monitoring system facilitates sterilization and is convenient and quick to use, thereby improving user experience.

In a first aspect of the present application, an insertion device is provided. The insertion device includes a housing assembly, a needle-advancing bracket, a fixing plate, and an insertion needle assembly.

An actuation button is disposed on the outer periphery side of the housing assembly.

The needle-advancing bracket is disposed in the housing assembly, where the needle-advancing bracket includes a sleeve, an accommodating slot, and at least two elastic claws disposed in the sleeve, a slot opening of the accommodating slot and a sleeve opening of the sleeve face away from each other, the accommodating slot is configured to accommodate a sensor assembly, the needle-advancing bracket is movable along a first direction and is capable of being limited in a first position and a second position in the housing assembly, the actuation button is capable of separating the housing assembly from the needle-advancing bracket so that the needle-advancing bracket moves from the first position to the second position, and a through hole is provided at the bottom of the sleeve.

The fixing plate is disposed in the housing assembly, where the fixing plate is capable of being locked to the at least two elastic claws, and the housing assembly is capable of separating the fixing plate from the at least two elastic claws.

The insertion needle assembly includes a needle hub and an insertion needle connected to the needle hub, where the needle hub is circumferentially clamped between the at least two elastic claws and is disposed between the at least two elastic claws and the bottom of the sleeve, the needle hub is movable toward the fixing plate, the insertion needle is capable of passing through the through hole and being engaged with the sensor assembly, and a sensor electrode of the sensor assembly extends through the insertion needle.

In some possible embodiments, a rib plate is disposed on the inner side of the housing assembly, a flange is provided on the outer periphery of the needle-advancing bracket, and when the needle-advancing bracket is located in the first position, the rib plate abuts against the flange.

In some possible embodiments, the insertion device further includes a first elastic member and a second elastic member.

The first elastic member is disposed in a first accommodating space formed by the sleeve and the housing assembly, where two ends of the first elastic member abut against the bottom of the sleeve and the housing assembly, respectively.

The second elastic member is disposed in a second accommodating space formed by the needle hub and the sleeve, where an opening of the needle hub faces the bottom of the sleeve, and two ends of the second elastic member abut against the bottom of the sleeve and the top of the needle hub, respectively.

In some possible embodiments, a first limit block and a second limit block are disposed in the housing assembly, the first limit block and the second limit block are spaced apart along the first direction, a first limit snap and a second limit snap are provided on the outer periphery of the sleeve, the first limit snap is engaged with the first limit block to cause the needle-advancing bracket to be located in the first position, the second limit snap is engaged with the second limit block to cause the needle-advancing bracket to be located in the second position, and the actuation button is capable of separating the first limit snap from the first limit block.

In some possible embodiments, the first limit snap is elastic along the radial direction of the sleeve, and the actuation button is capable of driving the first limit snap to move along the radial direction of the sleeve.

In some possible embodiments, one of the needle-advancing bracket and the housing assembly is provided with a guide groove, and the other of the needle-advancing bracket and the housing assembly is provided with a guide rib, where the guide rib is slidably connected to the guide groove.

In some possible embodiments, a step is provided on the inner side of an elastic claw, and the needle hub is disposed between the step and the bottom of the sleeve.

In some possible embodiments, the at least two elastic claws are engaged with the fixing plate, or the fixing plate is hinged to one of the at least two elastic claws.

In some possible embodiments, an elastic clamping arm is provided in the accommodating slot, and the sensor assembly is clamped by the elastic clamping arm.

In some possible embodiments, the housing assembly includes an upper housing and a top cover, and a protective cover, the top cover and the protective cover are detachably connected to two ends of the upper housing, the top cover is capable of separating the fixing plate from the at least two elastic claws, the actuation button is connected to the upper housing, and the protective cover is configured to face the accommodating slot.

In some possible embodiments, the insertion device further includes a first support base, where the first support base is fixedly connected to the upper housing, and the connection manner includes, but is not limited to, snap-fit connection, sliding groove connection, and adhesive dispensing.

In some possible embodiments, at least one guide column is disposed in the needle-advancing bracket, and the at least one guide column is configured to be mated with and connected to the fixing plate.

In some possible embodiments, the actuation button is provided with a pull ring, and the pull ring is configured to prevent a user from accidentally triggering the actuation button.

In a second aspect of the present application, an implantable analyte monitoring device is further provided. A sensor can be effectively mounted and fixed, thereby ensuring that the implantable analyte monitoring device can still maintain stable transmission of an electric signal after long-time use.

The implantable analyte monitoring device includes an upper cover and a lower housing connected to each other to form a housing, where a circuit board is mounted in the housing, and a signal input terminal of the circuit board is connected to a sensor through a socket.

The sensor includes an implant end and a signal connection end, where a fixing assembly configured to fix the signal connection end in the housing is disposed on the lower housing.

The socket includes a base and a terminal mounted on the base, the base is provided with an insertion slot configured to be engaged with the signal connection end, one end of the terminal extends into the insertion slot to contact a contact point on the signal connection end, and the other end of the terminal passes through the base to be electrically connected to the circuit board.

In some possible embodiments, contact points configured to transmit signals are provided on one or two side surfaces of the signal connection end.

In some possible embodiments, the fixing assembly includes an engagement slot portion, the engagement slot portion is located on the upper end surface of the lower housing and provided along the extension direction of the signal connection end, and the engagement slot portion is engaged with and fixes the bottom of the signal connection end.

In some possible embodiments, the fixing assembly includes clamping members, and at least one pair of the clamping members clamps and fixes the signal connection end.

In some possible embodiments, at least one limit slot corresponding to a clamping member is provided on two sides of the insertion slot, and the clamping member is inserted into the at least one limit slot.

In some possible embodiments, a communication portion configured to transmit a signal is provided between the signal connection end and the implant end, a pressing slot is provided between the communication portion and the signal connection end, and the pressing slot is engaged with the base.

In some possible embodiments, the terminal is integrally bent and formed, and the terminal includes a crimping portion connected to the contact point, a connection portion connected to the circuit board, and a conductive portion disposed between the crimping portion and the connection portion.

The crimping portion is a hook-shaped structure with an end portion inclined and extending toward the conductive portion.

In some possible embodiments, a second mounting slot is provided in the base, the conductive portion of the terminal is fixed in the second mounting slot, a third mounting slot communicating with the second mounting slot is provided at the top of the base, the connection portion of the terminal passes through the third mounting slot to be connected to the circuit board, a first mounting slot is provided between the second mounting slot and the insertion slot, the crimping portion of the terminal is disposed in the first mounting slot, and the outermost side of the crimping portion extends from the first mounting slot into the insertion slot to abut against and be connected to the contact point.

In some possible embodiments, one of the lower housing and the upper cover is provided with a first snap, the other of the lower housing and the upper cover is provided with a first snap slot corresponding to the position of the first snap, and the first snap snaps into the first snap slot such that the lower housing and the upper cover are engaged with each other in a snap-fit manner.

In some possible embodiments, the lower housing and the upper cover are engaged and sealed through adhesive dispensing or an elastic member.

In some possible embodiments, one of the lower housing and the upper cover is provided with a second snap, and the housing fixes the circuit board in the housing through the second snap.

In some possible embodiments, one of the lower housing and the upper cover is provided with the second snap, the second snap snaps into one of the upper end surface of the circuit board and the lower end surface of the circuit board, and the first snap supports the other of the upper end surface of the circuit board and the lower end surface of the circuit board.

In some possible embodiments, the lower housing is provided with positioning columns, the positioning columns are inserted into positioning holes of the circuit board to position the circuit board, and the lower housing and the upper cover are engaged with each other in a snap-fit manner to fix the circuit board.

In some possible embodiments, a hole in the upper cover is configured to allow an insertion needle hub to pass and be fixed therein, and a hole in the lower housing is configured to allow a needle cap to pass. The needle cap is detachably connected to the insertion needle hub to form a sterilization cavity. Both the insertion needle on the insertion needle hub and the implant end of the sensor extend into the sterilization cavity.

In a third aspect of the present application, an implantable analyte monitoring system is further provided. When the implantable analyte monitoring device is in use, the needle cap for sterilization can be quickly and effectively removed, thereby reducing the risk of bacterial infection. The implantable analyte monitoring system includes the insertion device according to the first aspect and the implantable analyte monitoring device according to the second aspect.

The implantable analyte monitoring device is provided with an insertion needle hub and a needle cap, and the implantable analyte monitoring device is mounted in the insertion device. The insertion device includes the upper housing, the top cover, and the protective cover detachably connected to the upper housing and the top cover. An applicator assembly configured to implement an insertion action and a retraction action is disposed in the insertion device, and the protective cover is connected to the needle cap for sterilization through a connector.

As an optional solution, the connector includes a base and a first snap member, and a fixing hole is provided in and penetrates through the protective cover. One end of the first snap member passes through the fixing hole to extend into the protective cover and be engaged with the upper end surface of a base plate of the protective cover in a snap-fit manner, and the other end of the first snap member is connected to the base. The base abuts against the lower end surface of the base plate of the protective cover.

As an optional solution, the connector further includes a second snap member. One end of the second snap member passes through the fixing hole to be engaged with the needle cap, and the other end of the second snap member is connected to the base.

### BRIEF DESCRIPTION OF DRAWINGS

FIG. 1 is an exploded view of an insertion device according to an embodiment of the present application.
FIG. 2A is a schematic view of an insertion device in the initial state according to an embodiment of the present application.
FIG. 2B is another schematic view of an insertion device in the initial state according to an embodiment of the present application.
FIG. 3 is a schematic view of an insertion device in the needle retraction state according to an embodiment of the present application.
FIG. 4 is a sectional view showing a needle-advancing bracket, a needle hub, and a fixing plate according to an embodiment of the present application.
FIG. 5 is a schematic view of a needle-advancing bracket according to an embodiment of the present application.
FIG. 6 is a schematic view showing the connection between a needle-advancing bracket and a fixing plate according to an embodiment of the present application.
FIG. 7 is a sectional view of an upper housing according to an embodiment of the present application.
FIG. 8 is a sectional view of a top cover according to an embodiment of the present application.
FIG. 9 is a schematic view of a sensor assembly and an insertion needle according to an embodiment of the present application.
FIG. 10 is a schematic view of a sensor assembly according to an embodiment of the present application.
FIG. 11 is another exploded view of an insertion device according to an embodiment of the present application.
FIG. 12 is another schematic view showing the connection between a needle-advancing bracket and a fixing plate according to an embodiment of the present application.
FIG. 13 is another schematic view of an insertion device in the needle retraction state according to an embodiment of the present application.
FIG. 14 is another sectional view showing a needle-advancing bracket, a needle hub, and a fixing plate according to an embodiment of the present application.
FIG. 15 is another schematic view of a needle-advancing bracket according to an embodiment of the present application.
FIG. 16 is a schematic view of an overall structure of an implantable analyte monitoring device according to an embodiment of the present application.
FIG. 17 is an exploded view of an implantable analyte monitoring device according to an embodiment of the present application.
FIG. 18A is a sectional view of an implantable analyte monitoring device according to an embodiment of the present application.
FIG. 18B is another sectional view of an implantable analyte monitoring device according to an embodiment of the present application.
FIG. 18C is a schematic view of the interior of an implantable analyte monitoring device according to an embodiment of the present application.
FIG. 19 is a structural view showing assembly of a lower housing and a sensor of an implantable analyte monitoring device according to an embodiment of the present application.
FIG. 20 is a structural view showing assembly of a socket and a sensor of an implantable analyte monitoring device according to an embodiment of the present application.
FIG. 21 is a structural view of a socket according to this embodiment.
FIG. 22 is a schematic view of a base and a terminal in a disassembled state according to an embodiment of the present application.
FIG. 23 is an exploded view showing a sealing assembly (in a dashed box) of an implantable analyte monitoring device according to an embodiment of the present application.
FIG. 24 is another exploded view of an implantable analyte monitoring device according to an embodiment of the present application.
FIG. 25 is a structural view of an implantable analyte monitoring device, an insertion needle, and a needle cap according to an embodiment of the present application.
FIG. 26 is another structural view showing assembly of a lower housing and a sensor of an implantable analyte monitoring device according to an embodiment of the present application.
FIG. 27 is another structural view showing assembly of a circuit board and a sensor of an implantable analyte monitoring device according to an embodiment of the present application.
FIG. 28 is another structural view of a socket according to an embodiment of the present application.
FIG. 29 is another exploded view showing the structure of a base, a terminal, and a fixing solder tab according to an embodiment of the present application.
FIG. 30 is a view showing the connection among a needle cap, a connector, and a protective cover according to an embodiment of the present application.
FIG. 31 is another structural view of an insertion device according to an embodiment of the present application.
FIG. 32 is a view showing the connection between a needle cap and a protective cover in an implantable analyte monitoring system according to an embodiment of the present application.
FIG. 33 is a structural view of a connector in an implantable analyte monitoring system according to an embodiment of the present application.
FIG. 34 shows enlarged details in region A in FIG. 32.
FIG. 35 is another schematic view showing some structures of a needle cap, a connector, and a protective cover according to an embodiment of the present application.

In the preceding figures:
- 1: housing assembly
- 110: rib plate
- 12: first limit block
- 13: second limit block
- 14: guide rib
- 15: hook claw
- 2: actuation button
- 21: pull ring
- 3: needle-advancing bracket
- 31: sleeve
- 32: accommodating slot
- 33: elastic claw
- 331: step
- 332: snap slot
- 34: through hole
- 35: flange
- 36: first limit snap
- 37: second limit snap
- 38: guide groove
- 39: elastic clamping arm
- 4: fixing plate
- 41: slot
- 42: snap
- 43: first connection portion
- 5: insertion needle assembly
- 51: needle hub
- 52: insertion needle
- 6: first elastic member
- 7: second elastic member
- A: first accommodating space
- B: second accommodating space
- 100: sensor assembly
- 101: transmitter
- 102: sensor electrode
- 201: first support base
- 301: guide column
- 11: implantable analyte monitoring device
- 111: upper cover
- 112: lower housing
- 1121: engagement slot portion
- 1122: clamping member
- 1123: first snap
- 1124: second snap
- 1125: mounting base
- 116: first sealing ring
- 1126: sealing groove
- 1127: second protrusion
- 113: sensor
- 1131: implant end
- 1132: signal connection end
- 1133: contact point
- 1134: pressing slot
- 1135: communication portion
- 1136: first protrusion
- 114: circuit board
- 1141: battery
- 1142: signal input terminal
- 115: socket
- 1151: base
- 1152: terminal
- 11521: crimping portion
- 11522: conductive portion
- 11523: connection portion
- 1153: insertion slot
- 1154: limit slot
- 1155: slot portion
- 1156: first mounting slot
- 1157: second mounting slot
- 1158: third mounting slot
- 1159: fixing solder tab
- 170: skin patch
- 180: needle cap plug
- 1000: implantable analyte monitoring system
- 121: upper housing
- 122: protective cover
- 1221: engagement seat
- 123: top cover
- 130: insertion needle hub
- 140: needle cap
- 142: connection groove
- 143: reinforcement rib
- 144: third protrusion
- 150: connector
- 151: base
- 152: first snap member
- 153: second snap member
- 16: applicator assembly

### DETAILED DESCRIPTION

Technical solutions of embodiments of the present application are described below in conjunction with the drawings. The embodiments described below are part, not all of the embodiments of the present application.

In the description of the present application, the terms "joined", "connected", and "fixed" are to be understood in a broad sense unless otherwise expressly specified and limited. For example, the term "connected" may refer to "fixedly connected", "detachably connected", or "integrated", may refer to "mechanically connected" or "electrically connected", or may refer to "connected directly", "connected indirectly through an intermediary", "connected inside two elements", or "an interaction relationship between two elements". Meanings of the preceding terms in the present application may be understood according to actual situations.

In the present application, unless otherwise expressly specified and limited, when a first feature is described as "on" or "under" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

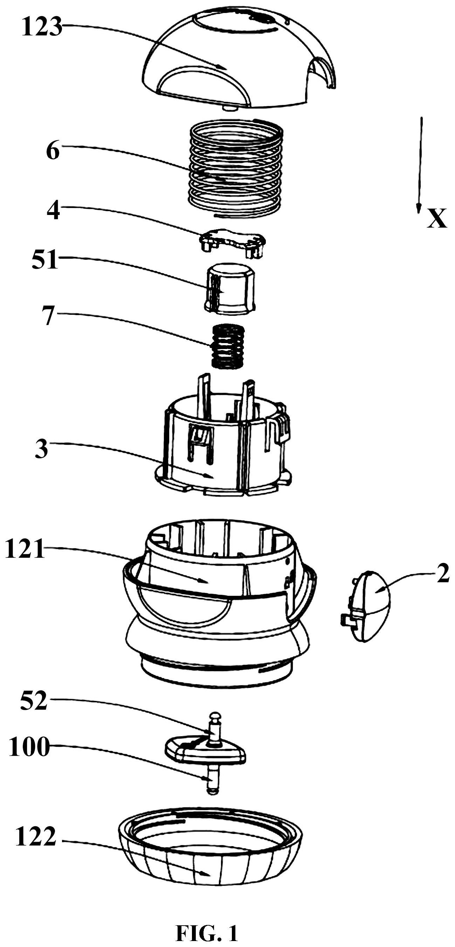

This embodiment provides an insertion device. As shown in FIGS. 1 to 3, the insertion device includes a housing assembly 1, an actuation button 2, a needle-advancing bracket 3, a fixing plate 4, and an insertion needle assembly 5. The actuation button 2 is disposed on the outer periphery side of the housing assembly 1. The needle-advancing bracket 3 is disposed in the housing assembly 1. The needle-advancing bracket 3 includes a sleeve 31, an accommodating slot 32, and at least two elastic claws 33 disposed in the sleeve 31. A slot opening of the accommodating slot 32 and a sleeve opening of the sleeve 31 face away from each other. The accommodating slot 32 is configured to accommodate a sensor assembly 100. The needle-advancing bracket 3 is disposed in the housing assembly 1. The needle-advancing bracket 3 is movable along a first direction and is capable of being limited in a first position and a second position in the housing assembly 1. The actuation button 2 is capable of separating the housing assembly 1 from the needle-advancing bracket 3 so that the needle-advancing bracket 3 moves from the first position to the second position. A through hole 34 is provided at the bottom of the sleeve 31. The fixing plate 4 is disposed in the housing assembly 1. The fixing plate 4 is capable of being locked to the elastic claws 33, and the housing assembly 1 is capable of separating the fixing plate 4 from the elastic claws 33. The insertion needle assembly 5 includes a needle hub 51 and an insertion needle 52 connected to the needle hub 51. The needle hub 51 is circumferentially clamped between the elastic claws 33 and is disposed between the elastic claws 33 and the bottom of the sleeve 31. The needle hub 51 is movable toward the fixing plate 4. The insertion needle 52 is capable of passing through the through hole 34 and being engaged with the sensor assembly 100. A sensor electrode 102 of the sensor assembly 100 extends through the insertion needle 52. In this embodiment, the first direction is the X direction.

The steps for using the insertion device are described below.

In the initial state: the needle-advancing bracket 3 is located in the first position away from the skin, the fixing plate 4 and the elastic claws 33 are locked, and the elastic claws 33 are constrained by the fixing plate 4. The sensor assembly 100 is disposed in the accommodating slot 32 of the needle-advancing bracket 3. The insertion needle 52 is capable of passing through the through-hole 34 and being engaged with the sensor assembly 100. The sensor electrode 102 of the sensor assembly 100 extends through the insertion needle 52.

In the actuated state: the actuation button 2 is actuated to separate the housing assembly 1 from the needle-advancing bracket 3, the needle-advancing bracket 3 moves from the first position away from the skin to the second position close to the skin, the sensor assembly 100 and the insertion needle 52 move to the second position along with the needle-advancing bracket 3, and the electrode of a sensor body is implanted subcutaneously along with the insertion needle 52. At the instant when the needle-advancing bracket 3 moves to the second position, the elastic claws 33 are separate from the fixing plate 4 so that the constraint on the elastic claws 33 is released, allowing the elastic claws 33 to elastically deform outward along the radial direction of the sleeve 31 and form a larger space; the needle hub 51 is no longer circumferentially constrained by the elastic claws 33 and moves toward the fixing plate 4 (that is, away from the skin), driving the insertion needle 52 to retract, and a skin patch fixed to the sensor body fixes the sensor assembly 100 to the surface of the skin. A user removes the insertion device, completing the implantation of the sensor assembly 100.

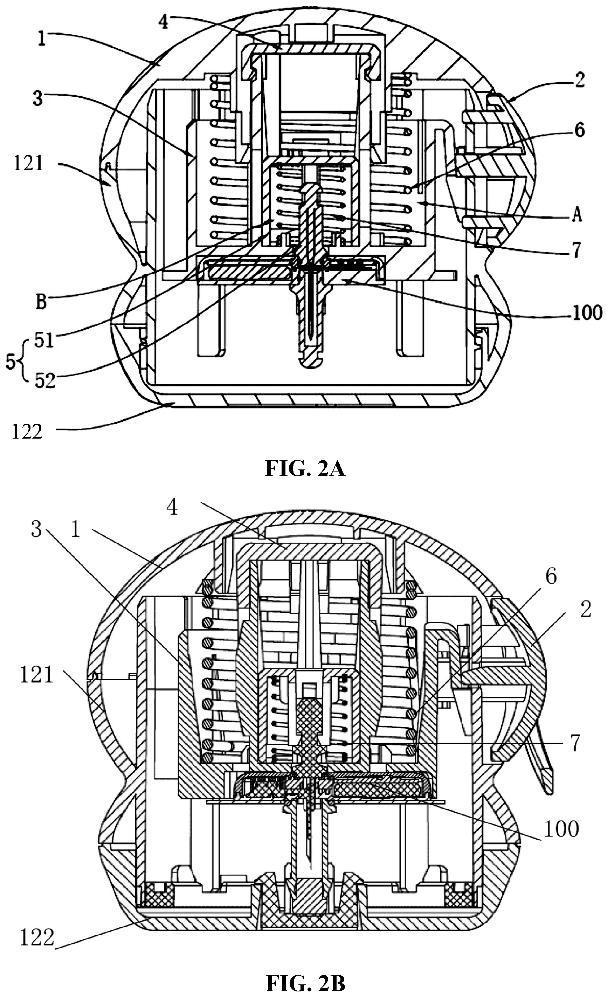

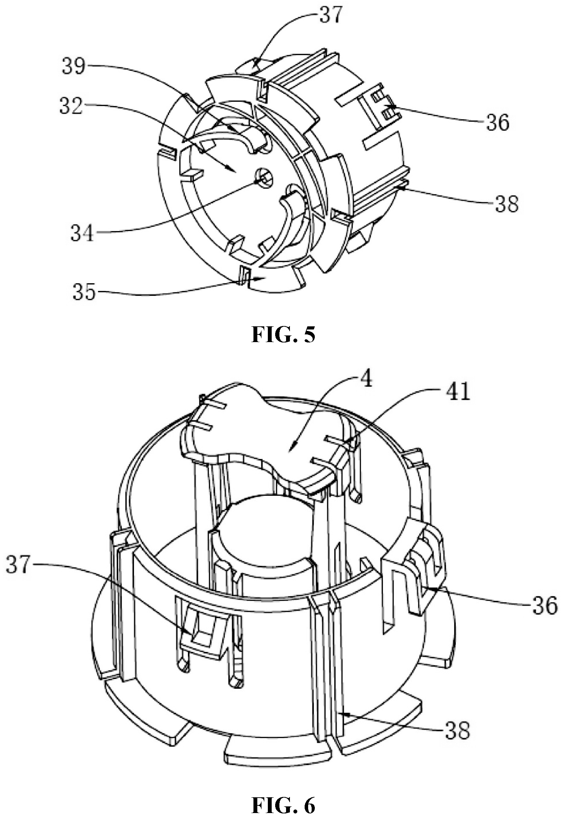

As shown in FIGS. 2A, 2B, and 5, the sensor assembly 100 is mounted in the accommodating slot 32, and the actuation button 2 is actuated so that the sensor assembly 100 can be implanted subcutaneously. This operation is convenient and improves user experience. The preceding structure is simple and compact, easy to manufacture, and low in cost. Compared with FIG. 2A, in FIG. 2B, the shape of an insertion needle hub 130 is changed, enabling the needle hub 51 to fix the insertion needle hub 130 more firmly.

As shown in FIGS. 2A and 2B to 7, a first limit block 12 and a second limit block 13 are disposed in the housing assembly 1. The first limit block 12 and the second limit block 13 are spaced apart along the first direction. A first limit snap 36 and a second limit snap 37 are provided on the outer periphery of the sleeve 31. In the initial state, the first limit snap 36 is engaged with the first limit block 12 to cause the needle-advancing bracket 3 to be located in the first position. After the actuation button 2 is pressed, the second limit snap 37 is engaged with the second limit block 13 to cause the needle-advancing bracket 3 to be located in the second position, preventing the needle-advancing bracket 3 from being separated from the housing assembly 1. The actuation button 2 is able to separate the first limit snap 36 from the first limit block 12, thereby separating the housing assembly 1 from the needle-advancing bracket 3. The needle-advancing bracket 3 moves until the second limit snap 37 limits and is engaged with the second limit block 13, limiting the needle-advancing bracket 3 in the second position in the housing assembly 1. The spacing between the first limit block 12 and the second limit block 13 along the first direction defines the movement range of the needle-advancing bracket 3.

In the initial state, the actuation button 2 abuts against a side surface of the first limit snap 36, and the bottom of the first limit snap 36 abuts against the first limit block 12. The first limit snap 36 is elastic along the radial direction of the sleeve 31 and is able to elastically deform inward. The actuation button 2 is able to drive the first limit snap 36 to move inward along the radial direction of the sleeve 31, separating the first limit snap 36 from the first limit block 12. Optionally, at least one first limit snap 36 and at least one first limit block 12 are provided and arranged in a one-to-one correspondence, and at least two second limit snaps 37 and at least two second limit blocks 13 are provided and arranged in a one-to-one correspondence.

The sleeve 31 is elastically connected to the housing assembly 1. The needle hub 51 is elastically connected to the elastic claws 33 and the bottom of the sleeve 31 along the first direction. Under the action of elasticity, the sleeve 31 and the needle hub 51 move along the first direction. For example, the insertion device further includes a first elastic member 6 and a second elastic member 7. The first elastic member 6 is disposed in a first accommodating space A formed by the sleeve 31 and the housing assembly 1. Two ends of the first elastic member 6 abut against the bottom of the sleeve 31 and the housing assembly 1, respectively. The second elastic member 7 is disposed in a second accommodating space B formed by the needle hub 51 and the sleeve 31. An opening of the needle hub 51 faces the bottom of the sleeve 31. Two ends of the second elastic member 7 abut against the bottom of the sleeve 31 and the top of the needle hub 51, respectively. In the initial state, the first elastic member 6 and the second elastic member 7 are each in a compressed state. In the actuated state, the needle-advancing bracket 3 and the housing assembly 1 are no longer constrained. A first spring returns to its original position, driving the needle-advancing bracket 3 to move from the first position to the second position. The needle hub 51 is released from the constraint of the elastic claws 33, and the opening of the second accommodating space B enlarges. The second elastic member 7 returns to its original position, driving the needle hub 51 to move away from the bottom of the sleeve 31 and driving the insertion needle 52 to retract. After the second elastic member 7 is restored from elastic deformation, the insertion needle assembly 5 is completely retracted. For example, each of the first elastic member 6 and the second elastic member 7 is a spring.

One of the needle-advancing bracket 3 and the housing assembly 1 is provided with a guide groove 38, and the other of the needle-advancing bracket 3 and the housing assembly 1 is provided with a guide rib 14. The guide rib 14 is slidably connected to the guide groove 38, thereby improving the movement accuracy of the needle-advancing bracket 3 along the first direction. Optionally, multiple guide grooves 38 and multiple guide ribs 14 are provided and arranged in a one-to-one correspondence. In this embodiment, the guide groove 38 is provided on the outer side of the sleeve 31, and the guide rib 14 is disposed on the inner side of the housing assembly 1.

A step 331 is provided on the inner side of each of the elastic claws 33, and the needle hub 51 is disposed between the step 331 and the bottom of the sleeve 31. When the elastic claws 33 are no longer constrained by the fixing plate 4, the opening formed among the multiple elastic claws 33 enlarges, the elastic claws 33 deform outward, and the step 331 is separated from the sleeve 31 along with the elastic claw 33.

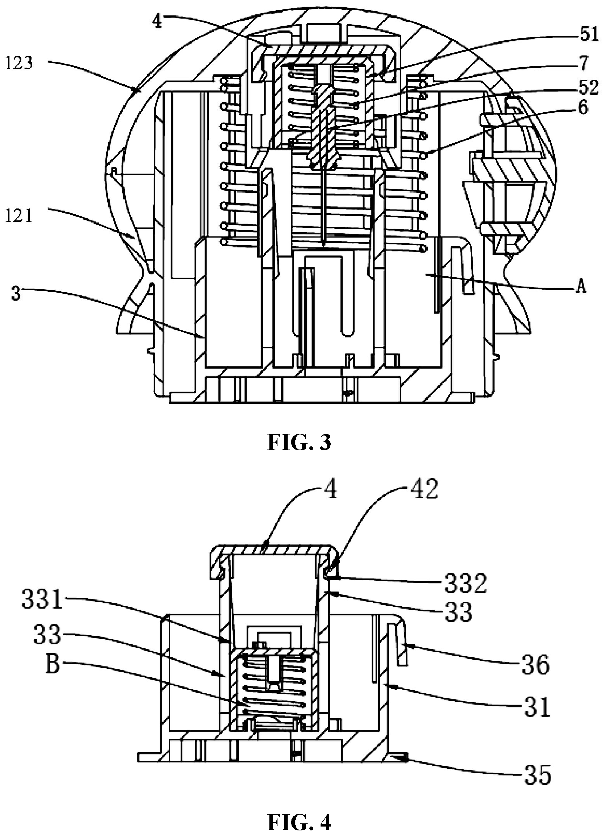

As shown in FIGS. 4, 6, and 8, the elastic claws 33 are engaged with the fixing plate 4, or the fixing plate 4 is hinged to one of the elastic claws 33. For example, the elastic claws 33 are provided with snap slots 332, and the fixing plate 4 is provided with snaps 42. The snaps 42 are engaged with the snap slots 332. The number of snaps 42 is equal to the number of snap slots 332, and the snaps 42 are in a one-to-one correspondence with the snap slots 332. As shown in FIG. 5, an elastic clamping arm 39 is provided in the accommodating slot 32, and the sensor assembly 100 is clamped by the elastic clamping arm 39, thereby improving connection reliability and preventing slippage.

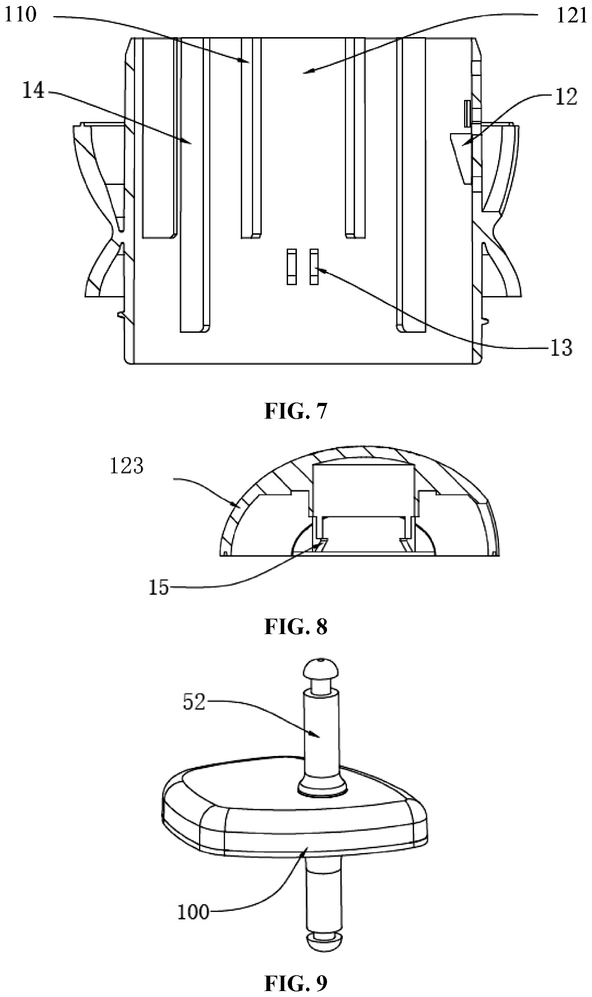

As shown in FIGS. 5 and 7, rib plates 110 are disposed on the inner side of the housing assembly 1, and a flange 35 is provided on the outer periphery of the needle-advancing bracket 3. In this solution, the flange 35 is provided on the outer periphery of the sleeve 31, and when the needle-advancing bracket 3 is located in the first position, the rib plates 110 abut against the flange 35. When the user pushes the needle-advancing bracket 3 toward the first position after completing the implantation action, the flange 35 of the needle-advancing bracket 3 abuts against the rib plates 110, thereby preventing the elastic claws 33 of the needle-advancing bracket 3 from re-engaging with the fixing plate 4 and avoiding reuse.

As shown in FIGS. 1 to 3, the housing assembly 1 includes an upper housing 121, a top cover 123, and a protective cover 122, and the top cover 123 and the protective cover 122 are detachably connected to two ends of the upper housing 121. The top cover 123 is capable of separating the fixing plate 4 from the elastic claws 33. The actuation button 2 is connected to the upper housing 121. The protective cover 122 is configured to face the accommodating slot 32. When the sensor assembly 100 is mounted in the accommodating slot 32, the protective cover 122 is removed so that the sensor assembly 100 is exposed externally. The upper housing 121 is open at the two ends. The top cover 123 and the protective cover 122 seal the two ends of the upper housing 121. The top cover 123 is provided with hook claws 15, and the fixing plate 4 is provided with slots 41. In an initial position, the fixing plate 4 is clamped between the elastic claws 33 and the top cover 123, and the fixing plate 4 and elastic claws 33 are locked through hinging or engagement. At the instant when the needle is inserted, the hook claws 15 hook the slots 41 so that the elastic claws 33 are separated from the fixing plate 4 and unlocked. Optionally, multiple hook claws 15 and multiple slots 41 may be provided. The number of hook claws 15 is equal to the number of slots 41, and the hook claws 15 are in a one-to-one correspondence with the slots 41. The upper housing 121 is provided with the first limit block 12, the second limit block 13, the guide rib 14, and the rib plates 110. The upper housing 121 adopts a split structure and is convenient to process and manufacture. The first direction is the direction of the centerline of the upper housing 121. Both the top cover 123 and the protective cover 122 are connected to the upper housing 121 through threads, snaps, or the like. The actuation button 2 is connected to the upper housing 121.

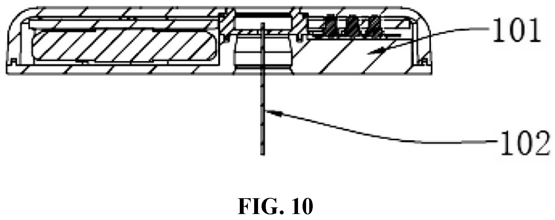

As shown in FIGS. 9 and 10, the sensor assembly 100 includes a transmitter 101 and the sensor electrode 102. The transmitter 101 is disposed in the accommodating slot 32 and detachably connected to the needle-advancing bracket 3. The sensor electrode 102 is provided with a portion disposed in the transmitter 101 and electrically connected to a circuit of the transmitter 101 and a portion protruding from the transmitter 101 and configured to be implanted subcutaneously. Reference may be made to the related art, and the details are not repeated.

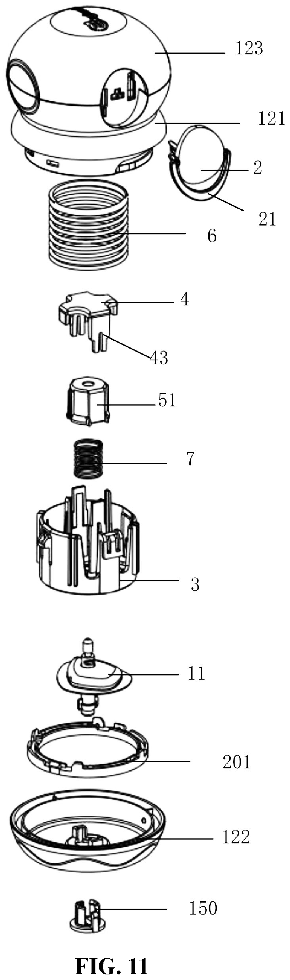

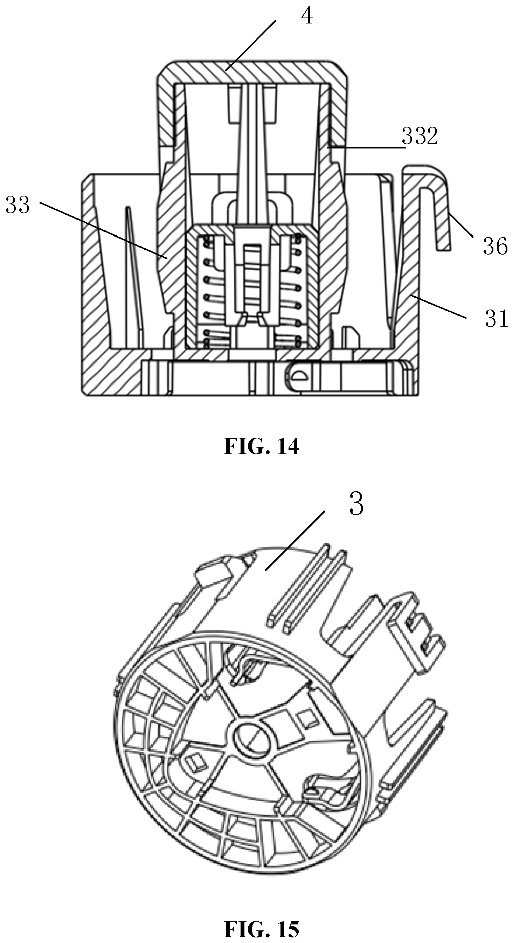

As shown in FIGS. 11 to 15, the present application further provides another example embodiment of the insertion device. The actuation button 2 may be provided with a pull ring 21. The pull ring 21 is configured to prevent the user from accidentally triggering the actuation button 2. That is, the actuation button 2 is a button for preventing accidental triggering. Before the insertion device is used, the actuation button 2 is able to be pressed only after the pull ring 21 is removed.

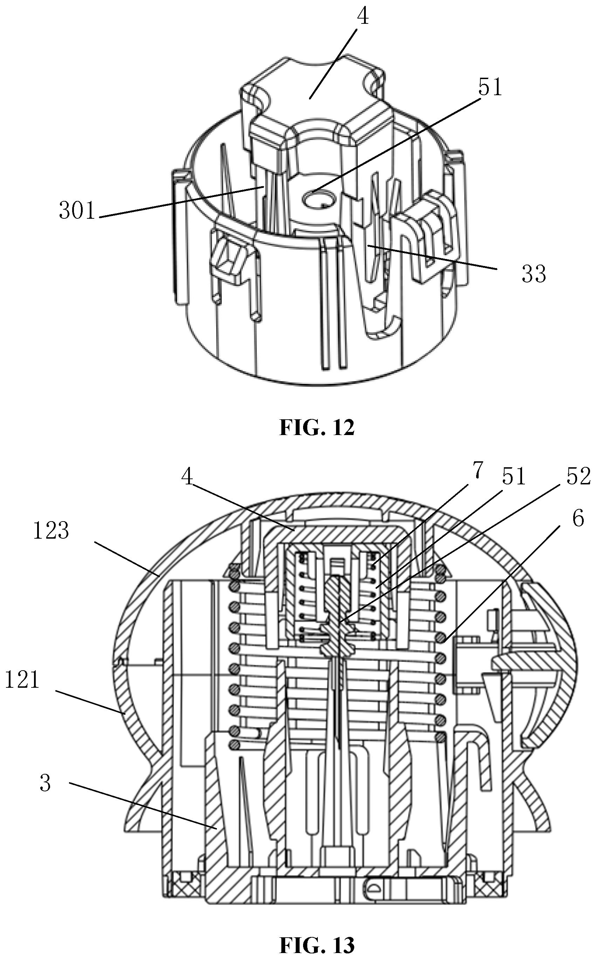

FIG. 11 shows a modified structure of the needle-advancing bracket 3. At least one guide column 301 may be disposed in the needle-advancing bracket 3. The guide column 301 is added to the existing elastic claw 33 (as shown in FIG. 12) so that the sliding path between the needle-advancing bracket 3 and the fixing plate 4 can be more reliably locked, thereby facilitating stable needle retraction.

Due to the addition of the guide column 301 to the needle-advancing bracket 3, the structure of the fixing plate 4 that is able to be locked to the needle-advancing bracket 3 also requires modification (as shown in FIG. 11). A connection structure that is capable of mating with the guide column 301 may be added to the fixing plate 4. For example, as shown in FIG. 12, a connection manner between the elastic claw 33 and the fixing plate 4 may be the same as that in FIGS. 4 and 6. The fixing plate 4 further includes at least one first connection portion 43 (as shown in FIGS. 11 and 12). The elastic claw 33 corresponding to the first connection portion 43 is disposed in the needle-advancing bracket 3. The first connection portion 43 may be connected to the elastic claw 33 through a sliding groove or a snap fit or may abut against the elastic claw 33 so that the stability of the connection between the needle-advancing bracket 3 and the fixing plate 4 is improved, thereby facilitating more precise positioning of the fixing plate 4 on the needle-advancing bracket 3. The number of the guide columns 301 and the number of the first connection portions 43 in FIGS. 11 and 12 may be set according to requirements and are not limited.

In this embodiment, as shown in FIG. 11, the insertion device may further include a first support base 201. The first support base 201 mates with and is connected to the upper housing 121. The first support base 201 is fixedly connected to the upper housing 121. The connection manner includes, but is not limited to, sliding groove connection, snap-fit connection, and adhesive bonding. The first support base 201 is provided so that the contact area between the insertion device and the skin can be reduced, thereby improving the implantation rate.

In one or more embodiments, a connector 150 is disposed at the bottom of the protective cover 122. The protective cover 122 is connected to a needle cap 140 (a sealing cap) through the connector 150, which can facilitate the removal of the needle cap 140.

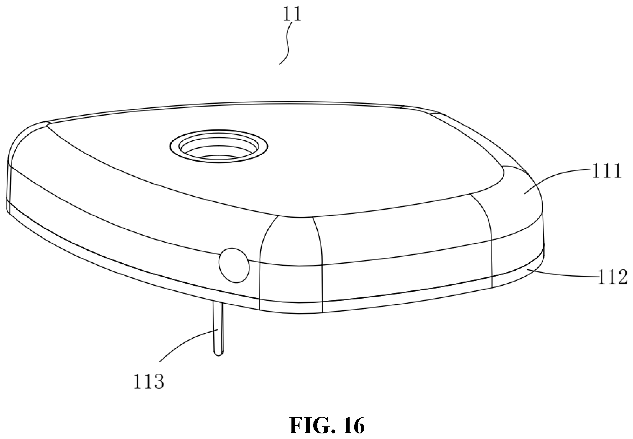

As shown in FIG. 16, this embodiment provides an implantable analyte monitoring device 11. This implantable analyte monitoring device 11 is mainly used for dynamic blood glucose monitoring, but is not limited thereto. The implantable analyte monitoring device 11 may also be used for monitoring other relevant targets such as lactate, acetylcholine, amylase, bilirubin, cholesterol, and chorionic gonadotropin.

As shown in FIG. 16, the implantable analyte monitoring device 11 is provided with an upper cover 111 and a lower housing 112. The upper cover 111 and the lower housing 112 are engaged with each other to form a housing. A circuit board 114 is mounted in the housing, and a signal input terminal 1142 of the circuit board 114 is connected to a sensor 113 through a socket 115. The sensor 113 includes an implant end 1131 and a signal connection end 1132. A fixing assembly configured to fix the signal connection end 1132 in the housing is disposed on the lower housing 112. The socket 115 includes a base 1151 and a terminal 1152 mounted on the base 1151. The base 1151 is provided with an insertion slot 1153 configured to be engaged with the signal connection end 1132. One end of the terminal 1152 extends into the insertion slot 1153 to contact a contact point 1133 on the signal connection end 1132, and the other end of the terminal 1152 passes through the base 1151 to be electrically connected to the circuit board 114.

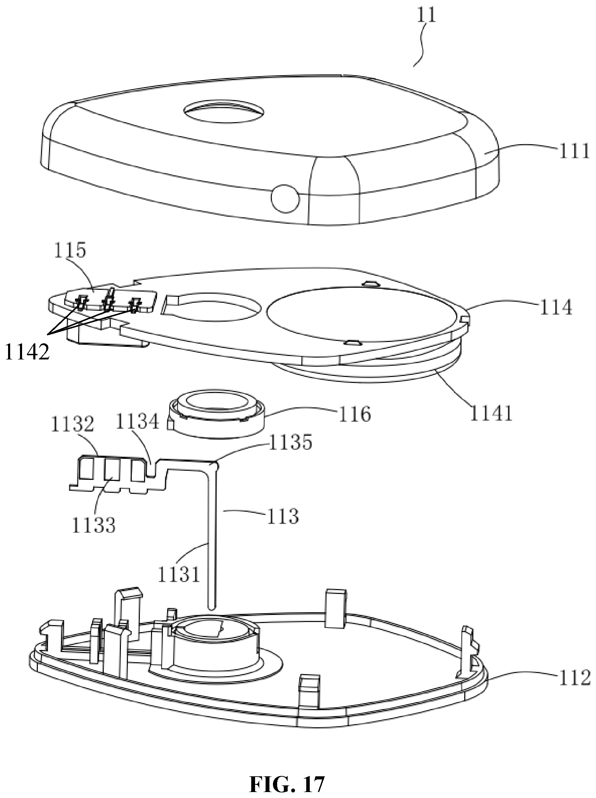

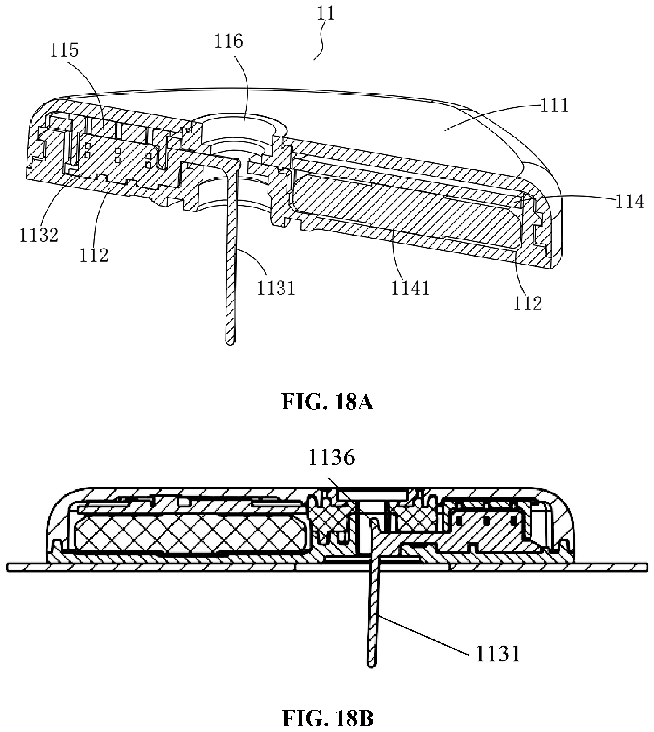

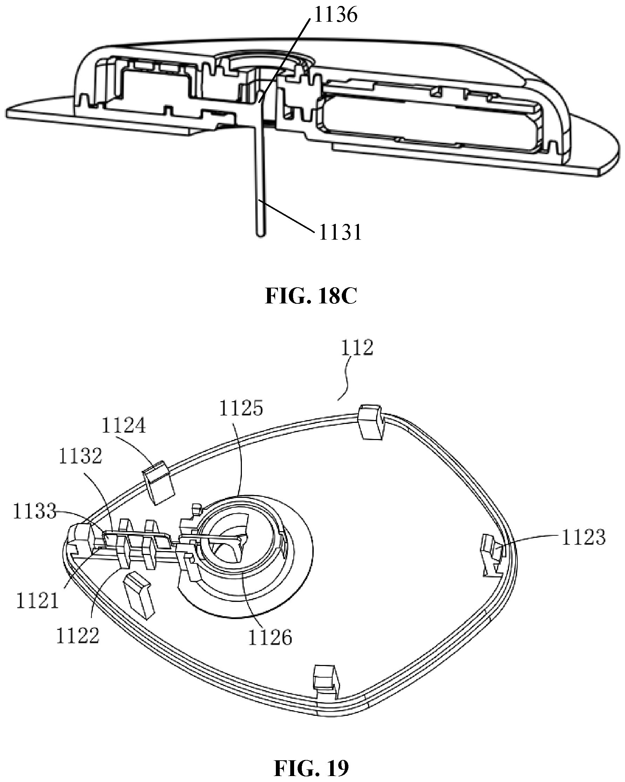

As shown in FIGS. 17, 18A, 18B, and 18C, the sensor 113 includes the implant end 1131 and the signal connection end 1132. A communication portion 1135 is provided between the implant end 1131 and the signal connection end 1132. The implant end 1131 is located outside the housing. During use, the implant end 1131 enters a subcutaneous tissue of a human body along with the insertion needle. Subsequently, the insertion needle quickly retracts from the subcutaneous tissue of the human body, while the implant end 1131 of the sensor 113 remains in the subcutaneous tissue of the human body to detect a glucose value of an interstitial fluid. A biological electrode on the implant end 1131 converts a biological signal for the glucose value of the interstitial fluid into an electric signal and transmits the electric signal to the signal connection end 1132 through the communication portion 1135. The signal connection end 1132 transmits the electric signal to the circuit board 114. A signal processing module and a signal transmission module are disposed on the circuit board 114. The signal processing module processes a collected signal, and the collected signal is sent to a user terminal, such as a mobile phone or a computer by the signal transmission module. The user may query monitoring data in real time through the terminal. In FIGS. 18B and 18C, a first protrusion 1136 may be provided at the top of the electrode of the sensor 113. Compared with the structure of the sensor 113 in FIG. 18A, the structure of the sensor 113 in FIGS. 18B and 18C is modified so that it can be convenient to position and mount the sensor 13 in the insertion needle 52.

In one or more embodiments, the communication portion 1135 configured to transmit the signal is provided between the signal connection end 1132 and the implant end 1131. A pressing slot 1134 is provided between the communication portion 1135 and the signal connection end 1132. The pressing slot 1134 is engaged with the base 1151.

In one or more embodiments, contact points 1133 configured to transmit signals are provided on one or two side surfaces of the signal connection end 1132.

In this embodiment, the signal connection end 1132 has a strip-shaped structure and is vertically disposed relative to the lower housing 112. The contact points 1133 electrically connected to the implant end 1131 may be provided on one or two side surfaces of the signal connection end 1132. Two contact points 1133 are typically provided. Alternatively, three contact points 1133 or another number of contact points 1133 may be provided. The specific number of contact points 1133 is determined based on the number of electrodes on the implant end 1131 and a function to be achieved.

As shown in FIG. 17, the socket 115 is mounted on the circuit board 114. The function of the socket 115 is to implement signal connection between the circuit board 114 and the sensor 113 and to further fix the sensor 113.

To adapt to the scenario in which the implantable analyte monitoring device 11 is able to continuously monitor data about an analyte of the human body for many days, especially to ensure that the user's daily activities such as work, life, and exercise are not affected during use of the implantable analyte monitoring device 11, relatively high requirements are imposed on the mounting and fixing of the sensor 113 and the stability of the signal.

To solve the mounting problem of the sensor 113, referring to FIG. 19, the fixing assembly includes an engagement slot portion 1121. The engagement slot portion 1121 is located on the upper end surface of the lower housing 112 and provided along the extension direction of the signal connection end 1132. The engagement slot portion 1121 is engaged with and fixes the bottom of the signal connection end 1132. A strip-shaped fixing rib is provided and protrudes along the upper end surface of the lower housing 112. The engagement slot portion 1121 is recessed along the extension direction of the fixing rib to match the specification of the signal connection end 1132. The engagement slot portion 1121 is engaged with the signal connection end 1132 to fix the bottom of the signal connection end 1132. Alternatively, the engagement slot portion 1121 may be directly provided on the lower housing 112 to be engaged with and fix the bottom of the signal connection end 1132.

With continued reference to FIG. 19, the fixing assembly further includes at least one pair of clamping members 1122. The at least one pair of clamping members 1122 is constituted by two clamping members 1122 disposed along two sides of the engagement slot portion 1121, respectively. The at least one pair of clamping members 1122 clamps and fixes the signal connection end 1132, thereby vertically fixing the middle and top portions of the signal connection end 1132.

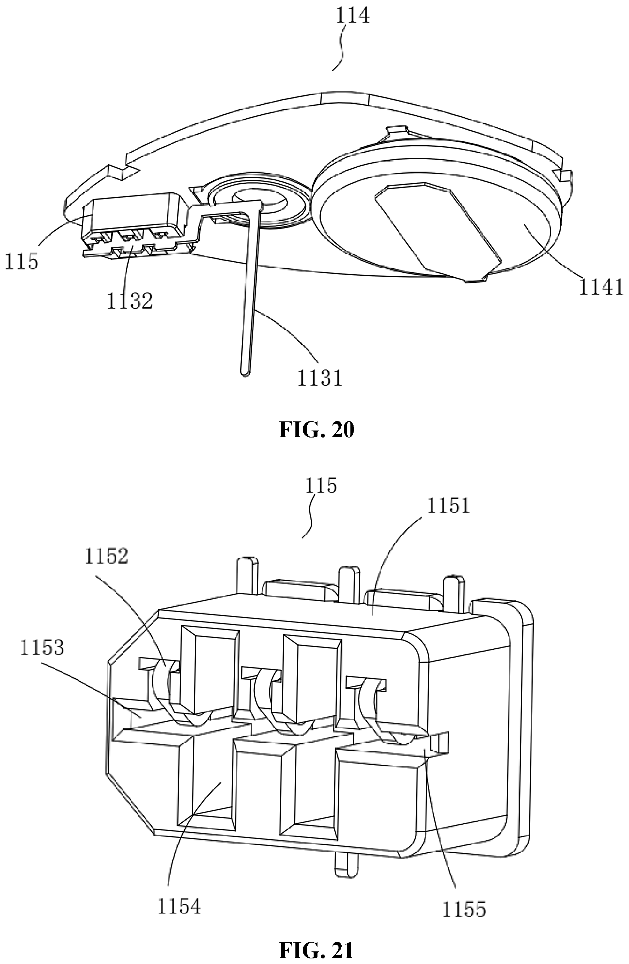

With continued reference to FIG. 19, a through hole is provided in the central position of the lower housing 112 and penetrates through the lower housing 112, where the through hole is configured to allow the insertion needle and the sensor 113 to pass. A mounting base 1125 is disposed at the outer edge of the through hole on the upper end surface of the lower housing 112. The mounting base 1125 is provided with a slot hole corresponding to the position of the through hole. The function of the slot hole is also to allow the sensor 113 and the insertion needle to pass. A sealing groove 1126 is recessed from the upper end surface of the mounting base 1125. The communication portion 1135 of the sensor 113 passes through the sealing groove 1126 to extend into the slot hole and be connected to the implant end 1131. During sterilization, to ensure the sealing effect of the implant end 1131, an adhesive is injected into the sealing groove 1126, thereby isolating the implant end 1131 of the sensor 113 from the signal connection end 1132 of the sensor 113. To solve the problem about the signal connection stability of the sensor 113, referring to FIG. 20, the signal connection between the sensor 113 and the circuit board 114 is implemented through the socket 115.

In one or more embodiments, at least one limit slot 1154 corresponding to the clamping members 1122 is provided on two sides of the insertion slot 1153, and the clamping member 1122 is inserted into the at least one limit slot 1154.

For example, as shown in FIG. 21, the socket 115 includes the base 1151 and terminals 1152. The base 1151 is made of an insulation material. The insertion slot 1153 is provided in the middle position of the bottom of the socket 115 along the length direction of the socket 115. The at least one limit slot 1154 corresponding in position and matching in specification with the clamping member 1122 is provided on the two sides of the insertion slot 1153. When the signal connection end 1132 of the sensor 113 is inserted into the socket 115, the signal connection end 1132 is inserted into and engaged with the insertion slot 1153, and the clamping member 1122 is simultaneously engaged and fixed in the limit slot 1154.

As shown in FIG. 21, a slot portion 1155 is provided on the sidewall of the base 1151 close to the implant end 1131. Correspondingly, as shown in FIG. 17, the pressing slot 1134 is provided between the communication portion 1135 and the signal connection end 1132. The width of the pressing slot 1134 is the same as the width of the sidewall of the base 1151. Thus, when the base 1151 is engaged with the sensor 113, the slot portion 1155 and the pressing slot 1134 are engaged with each other. Alternatively, the base 1151 may be provided with no slot portion 1155. In this case, the depth of the pressing slot 1134 needs to be increased so that the pressing slot can avoid the sidewall of the base 1151.

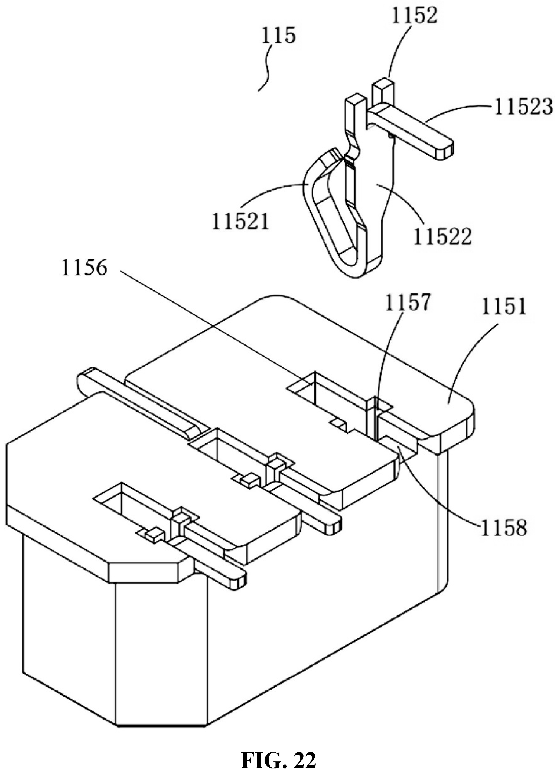

As shown in FIG. 22, the terminal 1152 may be integrally bent and formed from a conductive metal material such as copper, silver, or other electrically conductive metallic materials. The terminal 1152 includes a crimping portion 11521 connected to the contact point 1133, a connection portion 11523 connected to the circuit board 114, and a conductive portion 11522 disposed between the crimping portion 11521 and the connection portion 11523. The crimping portion 11521 is a hook-shaped structure with an end portion inclined and extending toward the conductive portion 11522. One end of the conductive portion 11522 is connected to the crimping portion 11521, and the other end of the conductive portion 11522 is connected to the connection portion 11523. The conductive portion 11522 transmits an electric signal from the crimping portion 11521 to the connection portion 11523. The connection portion 11523 is connected to the signal input terminal 1142 on the circuit board 114 and transmits the electric signal to the circuit board 114 for signal processing.

As shown in FIG. 22, the crimping portion 11521 is configured as the hook-shaped structure, and the hook tip of the crimping portion 11521 extends toward the conductive portion 11522. In the process where the crimping portion 11521 is press-fitted to the contact point 1133, the outermost position on the crimping portion 11521 relative to the conductive portion 11522 contacts the contact point 1133, and the contact point 1133 simultaneously compresses the outermost position on the crimping portion 11521, causing elastic deformation of the crimping portion 11521 toward the conductive portion 11522. Under the action of the elastic force, it can be ensured that the crimping portion 11521 can maintain a long-term stable contact with the contact point 1133, thereby improving signal stability.

As shown in FIGS. 21 and 22, a second mounting slot 1157 is vertically provided in the base 1151. The conductive portion 11522 of the terminal 1152 is fixed in the second mounting slot 1157. A third mounting slot 1158 communicating with the second mounting slot 1157 is provided at the top of the base 1151. The connection portion 11523 of the terminal 1152 passes through the third mounting slot 1158 to be soldered to the circuit board 114. In the soldering process, the connection portion 11523 is ensured to be connected to a signal input port of the circuit board 114, which can not only connect and fix the entire socket 115 to the circuit board 114 but also implement signal conduction between the socket 115 and the circuit board 114. A first mounting slot 1156 is provided between the second mounting slot 1157 and the insertion slot 1153 to ensure that the outermost position on the crimping portion 11521 can extend into the insertion slot 1153 to abut against the contact point 1133 on the signal connection end 1132. In one or more embodiments, the crimping portion 11521 of the terminal 1152 is disposed in the first mounting slot 1156, and the outermost side of the crimping portion 11521 extends from the first mounting slot 1156 into the insertion slot 1153 to abut against and be connected to the contact point 1133.

When irradiation sterilization is performed, since irradiation of electronic components by an electron beam may cause charge accumulation and further damage the electronic components, the circuit board 114 is not assembled in the housing during the irradiation sterilization. The circuit board 114 is assembled after the irradiation sterilization ends.

In one or more embodiments, one of the lower housing 112 and the upper cover 111 is provided with a first snap 1123, the other of the lower housing 112 and the upper cover 111 is provided with a first snap slot corresponding to the position of the first snap 1123, and the first snap 1123 snaps into the first snap slot such that the lower housing 112 and the upper cover 111 are engaged with each other in a snap-fit manner.

Optionally, the lower housing 112 and the upper cover 111 are engaged and sealed through adhesive bonding or an elastic member.

In one or more embodiments, either the lower housing 112 or the upper cover 111 is provided with a second snap 1124, and the housing fixes the circuit board 114 in the housing through the second snap 1124.

In one or more embodiments, the second snap 1124 snaps into either the upper end surface of the circuit board 114 or the lower end surface of the circuit board 114, and the first snap 1123 supports the other surface of the circuit board 114.

In one or more embodiments, a hole in the upper cover is configured to allow the insertion needle hub to pass and be fixed therein, and a hole in the lower housing is configured to allow the needle cap to pass. The needle cap is detachably connected to the insertion needle hub to form a sterilization cavity. Both the insertion needle on the insertion needle hub and the implant end of the sensor extend into the sterilization cavity.

To mount and fix the upper cover 111 and the lower housing 112, referring to FIG. 19, two or more first snaps 1123 are provided along the edge of the upper end surface of the lower housing 112. The sidewall of the upper cover 111 is provided with slot holes corresponding to the positions of the first snaps 1123. The first snaps 1123 snap into the slot holes of the upper cover 111 such that the lower housing 112 and the upper cover 111 are engaged with and connected to each other.

To mount and fix the circuit board 114, with continued reference to FIG. 19, multiple second snaps 1124 are provided along the edge of the upper end surface of the lower housing 112. The height difference between the second snap 1124 and the first snap 1123 is equal to or slightly less than the thickness of the circuit board 114. The lower end surface of the circuit board 114 is supported by the first snaps 1123, and the upper end surface of the circuit board 114 is limited by the second snaps 1124, thereby mounting and fixing the circuit board 114.

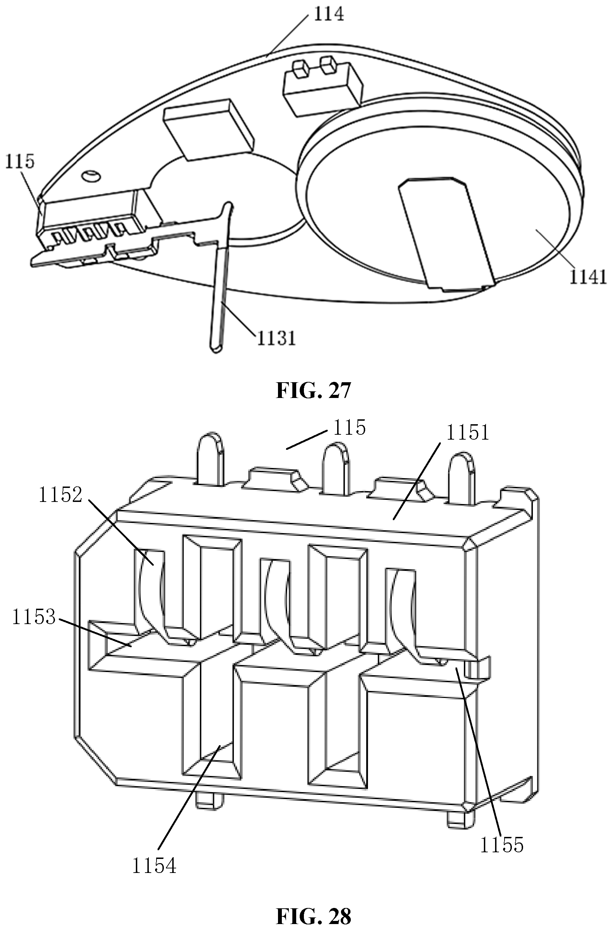

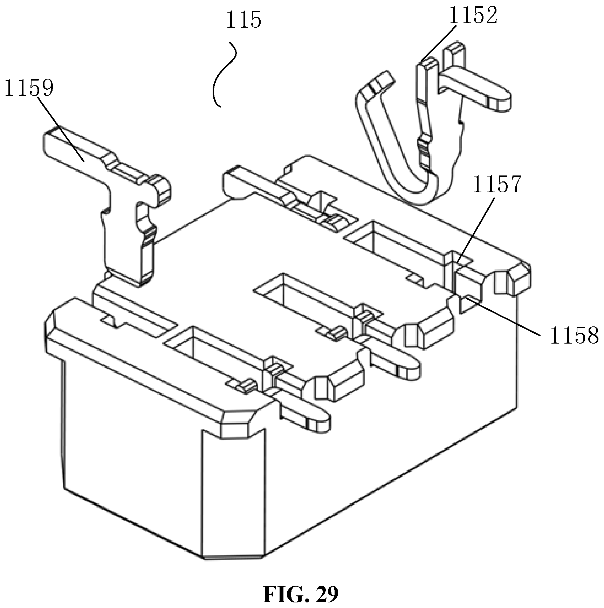

As shown in FIG. 29, the socket 115 may further include at least one fixing solder tab 1159. The fixing solder tab 1159 is inserted into the base 1151 through a corresponding mounting slot on the base 1151. The at least one fixing solder tab 1159 is disposed on the other side of the terminals 1152 to fix the socket 115 to the circuit board 114. For example, as shown in FIGS. 28 and 29, compared with the connection manner between the socket and the circuit board shown in FIGS. 20, 21, and 22, in addition to fixing the socket 115 to the circuit board 114 through the terminals 1152, the fixing solder tab 1159 is further provided so that the circuit board 114 can be mounted and fixed more stably.

As shown in FIG. 17, a battery 1141 is located on the lower end surface of the circuit board 114. This configuration aims to ensure that the battery 1141 and the socket 115 are located in the same direction so that the space occupied by the battery 1141 can be effectively saved. Thus, it is ensured that the internal layout of the entire implantable analyte monitoring device 11 is more reasonable, thereby reducing the size of the implantable analyte monitoring device 11.

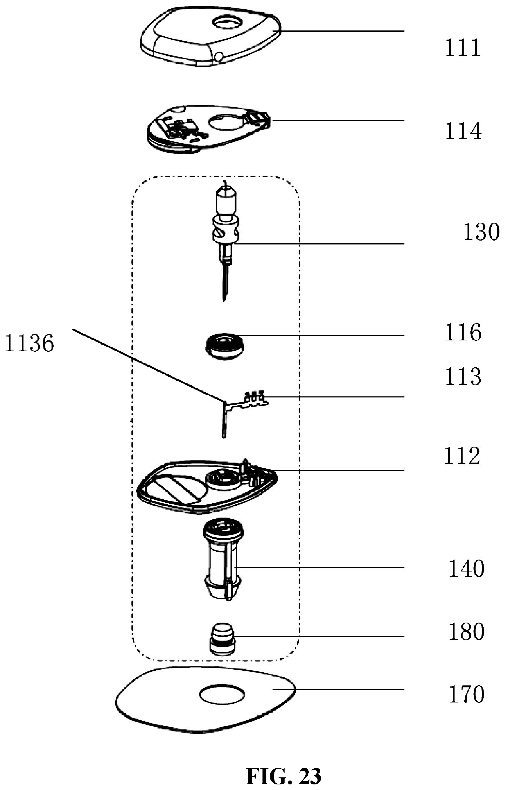

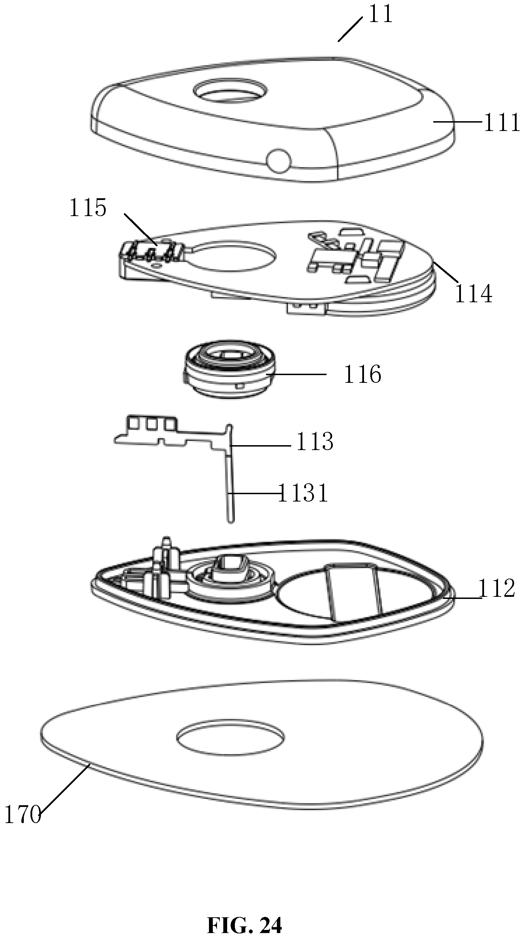

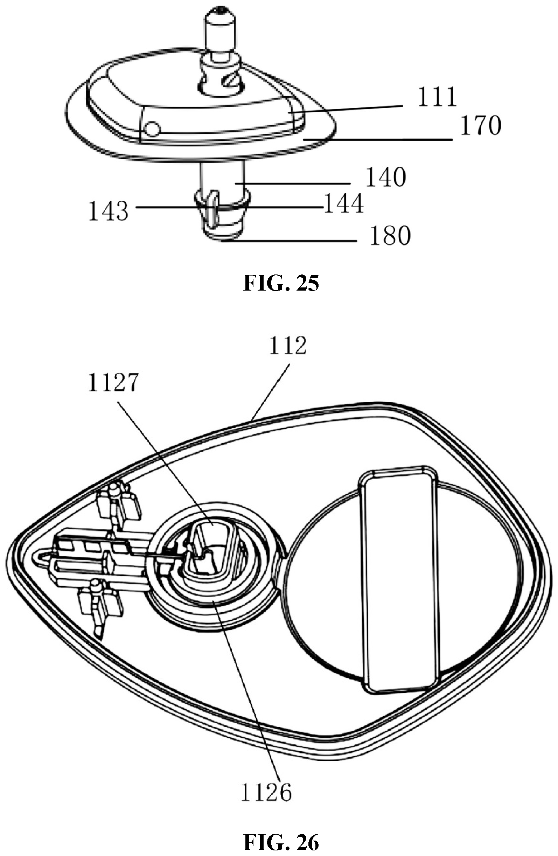

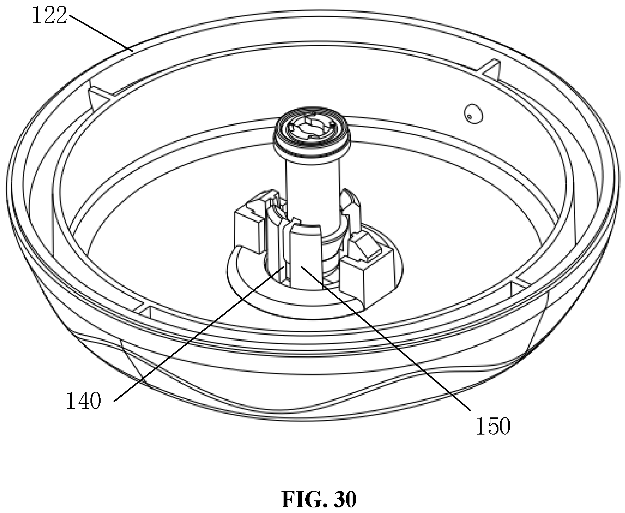

As shown in FIGS. 23 to 30, the present application further provides another embodiment of an implantable analyte monitoring device. In this embodiment, the shape of the insertion needle hub 130 is modified, as shown in FIG. 23. The shape of the insertion needle hub 130 is modified as shown in FIGS. 13 and 23. At least one slot is provided on the insertion needle hub 130 so that the needle hub 51 can fix the insertion needle hub 130 more firmly. One first protrusion 1136 (as shown in FIG. 23) may be provided at the top of the electrode of the sensor 113 to facilitate positioning with a hollow slot of the insertion needle.

In one or more embodiments, a second protrusion 1127 is disposed on the sealing groove 1126 to facilitate positioning with a first sealing ring 116.

In one or more embodiments, one or more third protrusions 144 are disposed at the bottom of the needle cap 140 to facilitate the connection between the needle cap 140 and the connector 150.

In one or more embodiments, a needle cap plug 180 is further disposed at the bottom of the needle cap 140. The needle cap plug 180 may be inserted into the needle cap 140 to facilitate machining of the needle cap 140.

A skin patch 170 may be further disposed at the bottom of the lower housing 112. The skin patch 170 is configured to contact the user's skin so that the device is better attached to the skin of the human body and is less likely to fall off.

This embodiment further includes the first sealing ring 116. The first sealing ring 116 can be overmolded on the second protrusion 1127, and the insertion needle and the first sealing ring 116 form a first seal.

A second sealing ring is disposed on the needle cap 140. The second sealing ring of the needle cap 140 is caused to abut against the bottom of the lower housing 112 through the engagement between the needle cap 140 and the insertion needle hub 130, thereby implementing a second seal.

The sensor of the implantable analyte monitoring device provided by the present application can be effectively mounted and fixed through the fixing assembly disposed on the lower housing. In addition, the socket further positions and fixes the sensor, and the signal connection between the sensor and the circuit board is implemented through the terminals on the socket. The device is applicable to a long-time use scenario of the implantable analyte monitoring device, prolongs the service life of the implantable analyte monitoring device, and improves signal transmission stability.

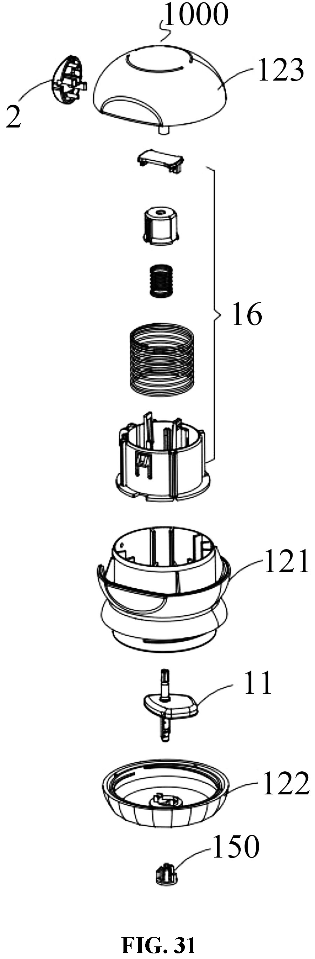

As shown in FIG. 31, this embodiment provides an implantable analyte monitoring system 1000. The implantable analyte monitoring system 1000 is mainly used for dynamic blood glucose monitoring, but is not limited thereto. The implantable analyte monitoring device 11 may also be used for monitoring other relevant targets such as lactate, acetylcholine, amylase, bilirubin, cholesterol, and chorionic gonadotropin. The implantable analyte monitoring device 11 in this embodiment is the sensor assembly 100 in the preceding embodiments, and the sensor 113 corresponds to the sensor electrode 102 in the preceding embodiments.

The implantable analyte monitoring system 1000 includes the preceding implantable analyte monitoring device. The implantable analyte monitoring device is provided with the insertion needle hub 130 and the needle cap 140 and is mounted in the insertion device. The insertion device includes a housing (that is, the upper housing 121 and the top cover 123) and the protective cover 122 detachably connected to the housing. The protective cover 122 is connected to the needle cap 140 for sterilization through the connector 150. An applicator assembly 16 configured to implement an insertion action and a retraction action is disposed in the housing.

As shown in FIG. 31, the monitoring system includes the housing and the protective cover 122 detachably connected to the housing. The applicator assembly 16 configured to implement the insertion action and the retraction action is disposed in the housing. The implantable analyte monitoring device 11 is disposed at the bottom of the applicator assembly 16.

As shown in FIG. 31, the applicator assembly 16 includes an insertion unit and a retraction unit. The insertion unit is configured to drive the insertion needle and the implant end 1131 into a patient's subcutaneous tissue after the actuation button 2 on the top cover 123 is pressed. The retraction unit retracts the insertion needle while the implant end 1131 remains in the patient's subcutaneous tissue. It is to be noted that the implant device is a conventional medical component in the medical field.

During the production and preparation of the monitoring device, sterilization operations need to be performed on the implant end 1131 of the sensor 113 and the insertion needle that will enter the human body, so as to prevent bacteria or viruses on the implant end 1131 and the insertion needle from causing infection upon entering the human body.

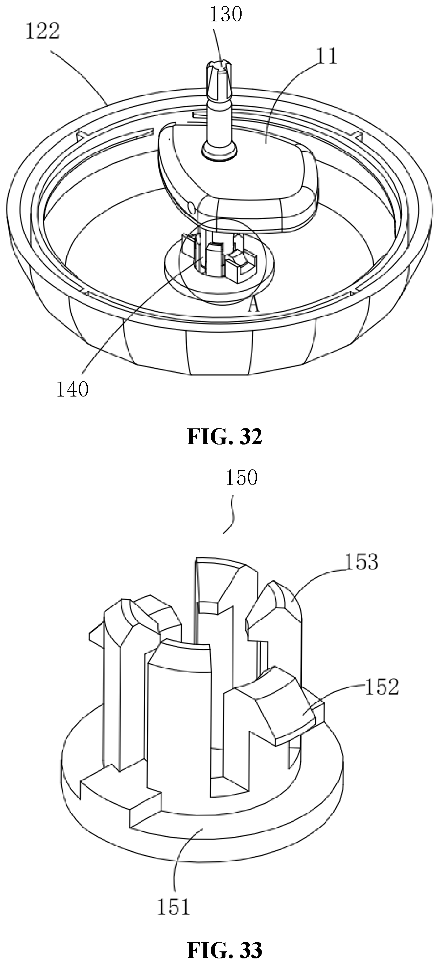

To implement the sterilization operations, as shown in FIGS. 31 and 32, the monitoring system is provided with the needle cap 140. The needle cap 140 is detachably connected to the insertion needle hub 130. The implant end 1131 and the insertion needle that require sterilization extend into the needle cap 140. The insertion needle hub 130, the mounting base 1125, the sealing adhesive injected into the sealing groove 1126, the lower housing 112, and the sealing ring collectively seal the open end of the needle cap 140, forming the sterilization cavity. The sterilization operations on the implant end 1131 and the insertion needle are completed in the sterilization cavity. Upon the completion of the irradiation sterilization, the bacteria and viruses on the implant end 1131 and the insertion needle are effectively eliminated, meeting standards for the implant end 1131 and the insertion needle to enter a human body. In addition, since the insertion needle and the implant end 1131 are sealed within the needle cap 140, the implant end 1131 and the insertion needle can be stored under sterile conditions.

During use, the needle cap 140 needs to be removed before insertion so that the insertion needle and the implant end 1131 are able to be exposed to air, thereby facilitating the entry of the insertion needle and the implant end 1131 into the subcutaneous tissue of the human body. If the needle cap 140 is removed manually, an improper operation may cause the insertion needle to puncture an operator's skin, posing a safety hazard and may also cause contamination of the insertion needle since the insertion needle is exposed to air. An improper operation may also cause the needle cap 140 to contact and collide with the insertion needle. In severe cases, the insertion needle may be deformed or bent, which poses significant risks to the entry of the insertion needle into the subcutaneous tissue of the human body. Therefore, how to quickly and effectively remove the needle cap 140 is also a problem to be solved by the monitoring system.

To solve the preceding problem about the removal of the needle cap 140, as shown in FIG. 32, in this embodiment, the needle cap 140 is fixed to the protective cover 122 through the connector 150. The protective cover 122 is connected to the bottom of the upper housing 121 through threads. The protective cover 122 may also be connected to the upper housing 121 through snaps or in other manners. When the protective cover 122 is removed from the upper housing 121, the protective cover 122 drives, through the connector 150, the needle cap 140 to separate from the implantable analyte monitoring device 11 together with the protective cover 122, implementing quick and effective detachment of the needle cap 140.

In one or more embodiments, the connector 150 includes a base 151 and a first snap member 152, and a fixing hole is provided in and penetrates through the protective cover 122. One end of the first snap member 152 passes through the fixing hole to extend into the protective cover 122 and be engaged with the upper end surface of a base plate of the protective cover 122 in a snap-fit manner, and the other end of the first snap member 152 is connected to the base 151. The base 151 abuts against the lower end surface of the base plate of the protective cover 122.

In one or more embodiments, the connector 150 further includes a second snap member 153. One end of the second snap member 153 passes through the fixing hole to be engaged with the needle cap 140, and the other end of the second snap member 153 is connected to the base 151.

For example, as shown in FIG. 33, the connector 150 includes the base 151, first snap members 152, and second snap members 153, and the fixing hole is provided in and penetrates through the protective cover 122. In this embodiment, the specification of the base 151 is greater than the specification of the fixing hole, thereby ensuring that the base 151 constantly remains outside the protective cover 122 and does not enter the interior of the protective cover 122 through the fixing hole.

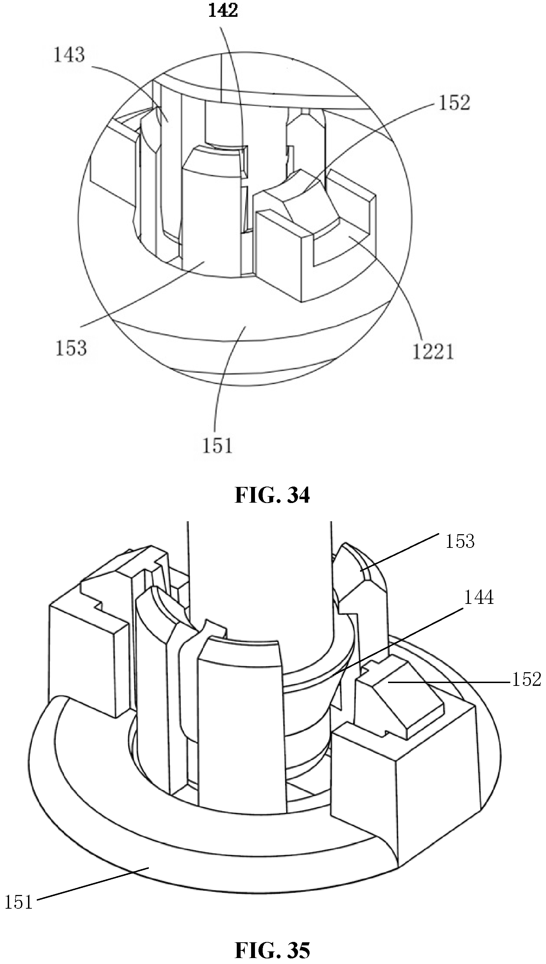

With continued reference to FIG. 34, hooks are provided at and protrude from the top ends of the first snap members 152 and the second snap members 153, so as to prevent relative misalignment between the connector 150 and the protective cover 122 during the removal of the protective cover 122 from the needle cap 140. At least one engagement seat 1221 corresponding to the first snap members 152 is provided on the outer side of the fixing hole of the protective cover 122. The bottom of the connector 150 abuts against the bottom of the protective cover 122 through the base 151. The top of the connector 150 is engaged with engagement seats 1221 through the snaps of the first snap members 152, thereby connecting the connector 150 to the protective cover 122 and effectively preventing the relative misalignment between the connector 150 and the protective cover 122.

With continued reference to FIG. 34, at least one reinforcement rib 143 is vertically disposed on the sidewall of the needle cap 140, so as to prevent the relative rotation between the connector 150 and the needle cap 140 during the removal of the protective cover 122 from the needle cap 140. Connection grooves corresponding in position and specification to the second snap member 153 are provided on two sides of the reinforcement rib 143, respectively. One end of the second snap member 153 passes through the fixing hole, extends into the protective cover 122, and is engaged with the connection grooves, thereby implementing the connection between the second snap member 153 and the needle cap 140. The reinforcement rib 143 can prevent the relative rotation caused by slippage between the second snap member 153 and the needle cap 140 during rotation.

In an embodiment, as shown in FIG. 35, the needle cap 140 may not include structures such as the reinforcement rib 143 and the connection grooves 142, and the needle cap 140 is fixed to the connector 150 through the third protrusion 144.

The implantable analyte monitoring system provided in the present application connects the needle cap to the protective cover through the connector. During use, the protective cover is removed, and the needle cap for sterilization can be simultaneously removed, thereby simplifying the operation, reducing the exposure risk, and improving the safety of the implantable analyte monitoring system.

The preceding embodiments of the present application are intended only to clearly describe the present application and not to limit implementations of the present application. Those of ordinary skill in the art may make changes or alterations in other different forms based on the preceding description. All embodiments do not need to be and cannot be exhausted herein. Any modifications, equivalent substitutions, and improvements that are made within the spirit and principle of the present application fall within the scope of the claims of the present application.

## Claims

1. An insertion device, comprising:
a housing assembly (1), wherein an actuation button (2) is disposed on an outer periphery side of the housing assembly (1);
a needle-advancing bracket (3) disposed in the housing assembly (1), wherein the needle-advancing bracket (3) comprises a sleeve (31), an accommodating slot (32), and at least two elastic claws (33) disposed in the sleeve (31), a slot opening of the accommodating slot (32) and a sleeve opening of the sleeve (31) face away from each other, the accommodating slot (32) is configured to accommodate a sensor assembly (100), the needle-advancing bracket (3) is movable along a first direction and is capable of being limited in a first position and a second position in the housing assembly (1), the actuation button (2) is capable of separating the housing assembly (1) from the needle-advancing bracket (3) so that the needle-advancing bracket (3) moves from the first position to the second position, and a through hole (34) is provided at a bottom of the sleeve (31);
a fixing plate (4) disposed in the housing assembly (1), wherein the fixing plate (4) is capable of being locked to the at least two elastic claws (33), and the housing assembly (1) is capable of separating the fixing plate (4) from the at least two elastic claws (33); and
an insertion needle assembly (5) comprising a needle hub (51) and an insertion needle (52) connected to the needle hub (51), wherein the needle hub (51) is circumferentially clamped between the at least two elastic claws (33) and is disposed between the at least two elastic claws (33) and the bottom of the sleeve (31), the needle hub (51) is movable toward the fixing plate (4), the insertion needle (52) is capable of passing through the through hole (34) and being engaged with the sensor assembly (100), and a sensor electrode (102) of the sensor assembly (100) extends through the insertion needle (52).

2. The insertion device according to claim 1, wherein a rib plate (110) is disposed on an inner side of the housing assembly (1), a flange (35) is provided on an outer periphery of the needle-advancing bracket (3), and when the needle-advancing bracket (3) is located in the first position, the rib plate (110) abuts against the flange (35).

3. The insertion device according to claim 1, further comprising:
a first elastic member (6) disposed in a first accommodating space (A) formed by the sleeve (31) and the housing assembly (1), wherein two ends of the first elastic member (6) abut against the bottom of the sleeve (31) and the housing assembly (1), respectively; and
a second elastic member (7) disposed in a second accommodating space (B) formed by the needle hub (51) and the sleeve (31), wherein an opening of the needle hub (51) faces the bottom of the sleeve (31), and two ends of the second elastic member (7) abut against the bottom of the sleeve (31) and a top of the needle hub (51), respectively.

4. The insertion device according to claim 1, wherein a first limit block (12) and a second limit block (13) are disposed in the housing assembly (1), the first limit block (12) and the second limit block (13) are spaced apart along the first direction, a first limit snap (36) and a second limit snap (37) are provided on an outer periphery of the sleeve (31), the first limit snap (36) is engaged with the first limit block (12) to cause the needle-advancing bracket (3) to be located in the first position, the second limit snap (37) is engaged with the second limit block (13) to cause the needle-advancing bracket (3) to be located in the second position, and the actuation button (2) is capable of separating the first limit snap (36) from the first limit block (12).

5. The insertion device according to claim 4, wherein the first limit snap (36) is elastic along a radial direction of the sleeve (31), and the actuation button (2) is capable of driving the first limit snap (36) to move along the radial direction of the sleeve (31).

6. The insertion device according to claim 1, wherein one of the needle-advancing bracket (3) and the housing assembly (1) is provided with a guide groove (38), and another of the needle-advancing bracket (3) and the housing assembly (1) is provided with a guide rib (14), wherein the guide rib (14) is slidably connected to the guide groove (38).

7. The insertion device according to claim 1, wherein a step (331) is provided on an inner side of an elastic claw of the at least two elastic claws (33), and the needle hub (51) is disposed between the step (331) and the bottom of the sleeve (31).

8. The insertion device according to claim 1, wherein the at least two elastic claws (33) are engaged with the fixing plate (4), or the fixing plate (4) is hinged to one of the at least two elastic claws (33).

9. The insertion device according to claim 1, wherein an elastic clamping arm (39) is provided in the accommodating slot (32), and the sensor assembly (100) is clamped by the elastic clamping arm (39).

10. The insertion device according to any one of claims 1 to 9, wherein the housing assembly (1) comprises an upper housing (121), a top cover (123), and a protective cover (122), the top cover (123) and the protective cover (122) are detachably connected to two ends of the upper housing (121), the top cover (123) is capable of separating the fixing plate (4) from the at least two elastic claws (33), the actuation button (2) is connected to the upper housing (121), and the protective cover (122) is configured to face the accommodating slot (32).

11. The insertion device according to claim 10, further comprising a first support base (201), wherein the first support base (201) is fixedly connected to the upper housing (121).

12. The insertion device according to any one of claims 1 to 9, wherein the actuation button (2) is provided with a pull ring (21), and the pull ring (21) is configured to prevent a user from accidentally triggering the actuation button (2).

13. The insertion device according to any one of claims 1 to 9, wherein at least one guide column (301) is disposed in the needle-advancing bracket (3), and the at least one guide column (301) is configured to be mated with and connected to the fixing plate (4).

14. An implantable analyte monitoring device, comprising an upper cover (111) and a lower housing (112) connected to each other to form a housing, wherein a circuit board (114) is mounted in the housing, and a signal input terminal (1142) of the circuit board (114) is connected to a sensor (113) through a socket (115);
wherein the sensor (113) comprises an implant end (1131) and a signal connection end (1132), wherein a fixing assembly configured to fix the signal connection end (1132) in the housing is disposed on the lower housing (112); and
the socket (115) comprises a base (1151) and a terminal (1152) mounted on the base (1151), the base (1151) is provided with an insertion slot (1153) configured to be engaged with the signal connection end (1132), one end of the terminal (1152) extends into the insertion slot (1153) to contact a contact point (1133) on the signal connection end (1132), and another end of the terminal (1152) passes through the base (1151) to be electrically connected to the circuit board (114).

15. The implantable analyte monitoring device according to claim 14, wherein contact points (1133) configured to transmit signals are provided on one or two side surfaces of the signal connection end (1132).

16. The implantable analyte monitoring device according to claim 14, wherein the fixing assembly comprises an engagement slot portion (1121), the engagement slot portion (1121) is located on an upper end surface of the lower housing (112) and provided along an extension direction of the signal connection end (1132), and the engagement slot portion (1121) is engaged with and fixes a bottom of the signal connection end (1132).

17. The implantable analyte monitoring device according to claim 14 or 16, wherein the fixing assembly comprises clamping members (1122), and at least one pair of the clamping members (1122) clamps and fixes the signal connection end (1132).

18. The implantable analyte monitoring device according to claim 17, wherein at least one limit slot (1154) corresponding to a clamping member of the clamping members (1122) is provided on two sides of the insertion slot (1153), and the clamping member (1122) is inserted into the at least one limit slot (1154).

19. The implantable analyte monitoring device according to claim 14, wherein a communication portion (1135) configured to transmit a signal is provided between the signal connection end (1132) and the implant end (1131), a pressing slot (1134) is provided between the communication portion (1135) and the signal connection end (1132), and the pressing slot (1134) is engaged with the base (1151).

20. The implantable analyte monitoring device according to claim 14, wherein the terminal (1152) is integrally bent and formed, and the terminal (1152) comprises a crimping portion (11521) connected to the contact point (1133), a connection portion (11523) connected to the circuit board (114), and a conductive portion (11522) disposed between the crimping portion (11521) and the connection portion (11523);
wherein the crimping portion (11521) is a hook-shaped structure with an end portion inclined and extending toward the conductive portion (11522).

21. The implantable analyte monitoring device according to claim 20, wherein a second mounting slot (1157) is provided in the base (1151), the conductive portion (11522) of the terminal (1152) is fixed in the second mounting slot (1157), a third mounting slot (1158) communicating with the second mounting slot (1157) is provided at a top of the base (1151), the connection portion (11523) of the terminal (1152) passes through the third mounting slot (1158) to be connected to the circuit board (114), a first mounting slot (1156) is provided between the second mounting slot (1157) and the insertion slot (1153), the crimping portion (11521) of the terminal (1152) is disposed in the first mounting slot (1156), and an outermost side of the crimping portion (11521) extends from the first mounting slot (1156) into the insertion slot (1153) to abut against and be connected to the contact point (1133).

22. The implantable analyte monitoring device according to claim 14, wherein one of the lower housing (112) and the upper cover (111) is provided with a first snap (1123), another of the lower housing (112) and the upper cover (111) is provided with a first snap slot corresponding to a position of the first snap (1123), and the first snap (1123) snaps into the first snap slot such that the lower housing (112) and the upper cover (111) are engaged with each other in a snap-fit manner.

23. The implantable analyte monitoring device according to claim 22, wherein one of the lower housing (112) and the upper cover (111) is provided with a second snap (1124), and the housing fixes the circuit board (114) in the housing through the second snap (1124).

24. The implantable analyte monitoring device according to claim 23, wherein the second snap (1124) snaps into one of an upper end surface of the circuit board (114) and a lower end surface of the circuit board (114), and the first snap (1123) supports another of the upper end surface of the circuit board (114) and the lower end surface of the circuit board (114).

25. The implantable analyte monitoring device according to any one of claims 14 to 24, wherein the communication portion (1135) configured to transmit the signal is provided between the signal connection end (1132) and the implant end (1131).

26. An implantable analyte monitoring system, comprising the implantable analyte monitoring device (11) according to any one of claims 14 to 25, wherein the implantable analyte monitoring device (11) is provided with an insertion needle hub (130) and a needle cap (140), and the implantable analyte monitoring device (11) is mounted in the insertion device according to any one of claims 1 to 13, wherein the insertion device comprises a housing and the protective cover (122) detachably connected to the housing, an applicator assembly (16) configured to implement an insertion action and a retraction action is disposed in the housing, and the protective cover (122) is connected to the needle cap (140) for sterilization through a connector (150).

27. The implantable analyte monitoring system according to claim 26, wherein the connector (150) comprises a base (151) and a first snap member (152), and a fixing hole is provided in and penetrates through the protective cover (122), wherein one end of the first snap member (152) passes through the fixing hole to extend into the protective cover (122) and be engaged with an upper end surface of a base plate of the protective cover (122) in a snap-fit manner, another end of the first snap member (152) is connected to the base (151), and the base (151) abuts against a lower end surface of the base plate of the protective cover (122).

28. The implantable analyte monitoring system according to claim 27, wherein the connector (150) further comprises a second snap member (153), one end of the second snap member (153) passes through the fixing hole to be engaged with the needle cap (140), and another end of the second snap member (153) is connected to the base (151).
